# EUROPEAN PATENT APPLICATION

(11) **EP 4 566 532 A1**
(43) Date of publication of application: **11.06.2025**
(21) Application number: 23214024.4
(22) Date of filing: 04.12.2023
(51) Int. Cl.: A61B 5/11, A61B 5/113

(54) **INFANT MONITORING**

(71) Applicant: Koninklijke Philips N.V., 5656 AG Eindhoven (NL)
(72) Inventor: GOSENSHUIS, Daan Hendrik, Eindhoven (NL); BERNTSEN, Luc, Eindhoven (NL); SINGH, Jetendra Kumar, Eindhoven (NL)
(74) Representative: Philips Intellectual Property & Standards

(57) **Abstract**

Proposed concepts thus aim to provide schemes, solutions, concepts, designs, methods, and systems pertaining to determining if an infant is present in a monitored target area. In particular, embodiments aim to provide a method for detecting the presence or absence of an infant from a target area by processing at least one image of the target area using both an object detection model and a movement detection model.

## Description

### FIELD OF THE INVENTION

This invention relates to the field of infant monitoring, and in particular to determining the absence of an infant from a monitored area.

### BACKGROUND OF THE INVENTION

Infant monitoring systems are often used by parents and carers to monitor an infant whilst they sleep, allowing the parent/carer to check in on the sleeping infant remotely. Infant monitoring systems are often provided with one or more sensors to take visual and/or audio data of a monitored area which may then be relayed to the parent via a separate device or via an app on the parent's smart phone or other personal smart device.

Modern infant monitoring systems may be provided with additional functionalities that track and log the sleep periods of an infant. They may provide additional reassurance or insights to parents on the quantity and quality of sleep their infant is getting. There is therefore a need for a robust method for analysing data from an infant monitoring system to confidently determine whether a sleeping infant is absent from a monitored bed area.

### SUMMARY OF THE INVENTION

The invention is defined by the claims.

According to an aspect of the invention, there is provided a method for detecting the presence or absence of an infant from a monitored target area. The method comprises: processing first image data of the monitored target area with an object detection model to determine a presence status of the infant, the presence status being indicative of whether the infant is present in the target area; independent of processing the first image data, processing second image data of the monitored target area with a movement detection model to determine a breathing status of the infant, the breathing status describing at least one breathing parameter of the infant; and determining, based on the determined presence status of the infant and the determined breathing status of the infant, a value indicating whether the infant is present in the target area.

Proposed concepts thus aim to provide schemes, solutions, concepts, designs, methods, and systems pertaining to determining if an infant is present in a monitored target area. In particular, embodiments aim to provide a method for detecting the presence or absence of an infant from a target area by processing at least one image of the target area using both an object detection model and a movement detection model.

In other words, it is proposed that image data of a monitored area may be independently analysed using both an object detection model and a movement detection model to determine both a likelihood that there is an infant present in an image of the target area, and parameters relating to the breathing of the infant. These two pieces of information are then used together to determine whether the infant is absent from a target area. This may, for example, facilitate the accurate and/or automatic logging of infant sleep periods. It may also enable the provision of warnings to parents/carers if the infant is suddenly or unexpectedly absent from their crib.

It is proposed that both movement detection models and object detection models may be used in conjunction with each other to determine the likelihood of an infant being absent from a target area from image data. In this way, both information on the likelihood of an infant being present in an image of the target area and information on the breathing of the infant can be used together to determine the likelihood of the infant being absent.

The proposed method therefore leverages both infant breathing status information, gathered by detecting movement in image data of the target area, and infant presence status data, gathered by detecting the presence of an infant in image data of the target area, to achieve an accurate and robust determination of the likelihood of the infant being present in or absent from the target area.

In the proposed method the two processing steps, processing first image data with an object detection model and processing second image data with a movement detection model, are carried out independently. By this it is meant that the presence status is always determined, irrespective of the determined value of the breathing status. Similarly, the breathing status is always determined irrespective of the value of the presence status. In this way, both pieces of information are always used in the determination of whether the infant is present or absent from the target area which may result in more accurate determinations of infant absence.

It will also be appreciated that since the second image data is always processed with a movement detection model, irrespective of the value of the presence status, it is possible to carry out the two image processing steps in any sequential order, at overlapping times, or in parallel for example. Carrying out the two processing steps in parallel may result in a quicker determination of infant presence or absence than a method in which the steps must be carried out sequentially. The proposed method thus easily allows for real-time monitoring of an infant in monitored target crib area.

By always using both presence status data and breathing status data to determine whether the infant is absent from the target area, the confidence level of any resulting presence or absence determinations may be high. An improved method for detecting the absence or presence of an infant in a target area may therefore be provided by proposed concepts.

Using only an AI object detection model on image data to detect the presence of a baby in a bed or crib can often lead to falsely determining the infant is absent. These models may be trained to detect a particular body part of the infant. If infant is moving significantly or partially covered by a blanket or toy, the model may struggle to detect the infant even when they are present in the target area. Thus, using only the outcome of processing an image of the target area with an object detection model to determine if the infant is absent may lead to frequent false infant absence detections.

The proposed invention describes a method of detecting whether an infant is in their crib based on processing one or more captured images of the crib with both an object detection model and a motion detection model. Using both models in conjunction with each other may provide a detection method that results in fewer false absence detections. In addition, the method may be robust/agnostic to an infant's coverage by garments/blankets as these factors which may influence the accuracy of an object detection only model will not affect the determinations of the motion detection model.

Further, since the proposed detection method is based simply on processing image data, embodiments may support detecting whether an infant is absent from a target area without any specialised hardware i.e., based on images from a camera-based infant monitor, webcam, or smartphone.

Ultimately, an improved method for detecting the presence or absence of an infant from a target area may be supported by the proposed concept(s).

In some embodiments, the presence status may comprise a probability value. In this way, the presence status may provide quantitative information on the likelihood of an infant being present in the target area.

In some embodiments, the at least one breathing parameter of the infant may describe at least one of: a probability that a breathing infant is present in the target area; a respiration rate; a respiration depth; and a respiration regularity parameter. Therefore, the motion detection model may be configured to determine information about both the presence of a breathing infant and the quality of the detected breathing of the infant. Both of these factors may be of use in determining whether the infant is absent from the target area. For example, irregularities in the detected breathing may correspond to real irregularities in the breathing of the infant but alternatively may indicate incorrect allocation of detected motion to breathing of the infant and therefore information describing these irregularities may influence a resulting determination of the likelihood of an infant being absent from the target area.

In some embodiments, determining a value indicating whether the infant is present in the target area may comprise: comparing the presence status to a first threshold; comparing the breathing status to a second threshold; and determining, based on the comparison of the presence status to the first threshold and the breathing status to a second threshold, a value indicating whether the infant is present in the target area. In this way, predefined ranges may be provided to define the logic of the presence/absence determination. For example, a determination of infant absence may require both the presence status and the breathing status to lie within a certain range.

In some embodiments, the object detection model may be trained to detect at least one body part of the infant. In this way, the object detection model determines the likelihood of the infant being present in the first image data by identifying the presence of infant body parts in the image data. The body part may, for example, be the head of the infant as this is the body part most usually uncovered by blankets and therefore detection of an infant head is the most reliable metric for infant presence.

In some embodiments, the second image data of the monitored target area may comprise a plurality of time-sequenced images. This allows for the detection of movement that occurs in the time intervals between each of the time-sequenced images by detecting changes in the image data over time.

In some embodiments, processing a plurality of time-sequenced images of the monitored target area with a movement detection model may comprise detecting periodic movement. Motion related to the breathing of the infant is most likely to be detected as periodic motion and therefore by detecting periodic movement the model is able to identify detected movement that may relate to breathing of the infant.

In some embodiments, detecting periodic movement may further comprise determining at least one of: the periodicity of the periodic movement; the amplitude of the periodic movement; and the regularity of the periodic movement. These parameters describing the periodic motion may then correspond to various breathing parameters of the infant.

The first image data and the second image data may in some embodiments be from a single image sensor, thereby removing the need to have multiple sensors monitoring the target area.

In some embodiments, there is provided a computer program comprising code means for implementing any of the methods described above when said program is run on a processing system.

According to another aspect of the invention, there is provided a processor arrangement configured to run a computer program according to a proposed embodiment.

According to another aspect of the invention, there is provided a system for detecting the absence of an infant from a monitored target area, the system comprising an image processing unit configured to run the computer program described above.

In some embodiments, the system or processor arrangement described above may further comprise an output interface configured to receive, from the system or processor arrangement, and display an indication of whether the infant is absent from the target area.

The system may be remotely located from a user device for monitoring an infant. In this way, a user (such as a parent or carer) may have an appropriately arranged system that can receive information at a location remotely located from the system for determining the presence of an infant in a monitored target area. Embodiments may therefore enable a user to detect the presence or absence of the infant from the target using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system for detecting the presence or absence of an infant from a monitored target area; and a client device comprising an outputinterface. Dedicated data processing means may therefore be employed for the purpose of detecting the presence or absence of an infant from a target area, thus reducing processing requirements or capabilities of other components or devices of the system.

The system may further include a client device, wherein the client device comprises the image processing unit and a display unit. In other words, a user (such as a parent or carer) may have an appropriately arranged client device (such as a laptop, tablet computer, mobile phone, PDA, etc.) which processes received image data in order to detect the absence of an infant from a monitored target area being. Purely by way of example, embodiments may therefore provide a monitoring or observation system that enables monitoring of one or more infants from a single location, wherein real-time communication between an infant and monitoring user (e.g. parent or carer) is provided and can have its functionality extended or modified according to proposed concepts, for example.

It will be understood that processing capabilities may therefore be distributed throughout the system in different ways according to predetermined constraints and/or availability of processing resources.

Thus, another aspect of the invention may provide an infant monitoring system that comprises: a system for detecting the presence or absence of an infant from a monitored target area according to a proposed embodiment; and at least one of: an output interface configured to output an indication of whether an infant is absent from the target area; an input interface configured to permit to user of the infant monitoring system to select the target area; and an image sensor. In this way the image sensor and the system or processing arrangement used to process the image data may be present in a single device along with an interface that displays to the user the area being monitored by the image sensor and allows the user to select on the image a target area. This target area may correspond to the mattress of the bed or crib being monitored and, in this way, the system may be configured to detect whether an infant is absent from their bed/crib.

Embodiments may be employed in combination with conventional/existing infant monitoring methods and systems. In this way, embodiments may integrate into legacy systems to improve and/or extend their functionality and capabilities. An improved infant monitoring system may therefore be provided by proposed embodiments.

Thus, there may be proposed concepts for detecting the absence of an infant from a monitored target area, and this may be done based on processing an image of the target area with both an object detection model and a movement detection model. These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 depicts a simplified flow diagram of a method for detecting the presence or absence of an infant from a target area being monitored by an image sensor according to a proposed embodiment;
Fig. 2 depicts a simplified flow diagram of a method for processing image data with a movement detection model according to a proposed embodiment;
Fig. 3 is a simplified block diagram of a system for detecting the presence or absence of an infant from a target area according to a proposed embodiment; and
Fig. 4 illustrates a simplified block diagram of an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art of practicing the claimed invention, from a study of the drawings, the disclosure, and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to detecting the presence or absence of an infant from a monitored target area (for example, the mattress area of a cot/crib being monitored by a video infant monitor). According to proposed concepts, several possible solutions may be implemented separately or jointly. That is, although these possible solutions may be described below separately, two or more of these possible solutions may be implemented in one combination or another.

Embodiments of the invention aim to provide a concept for automatically determining whether an infant is present or absent from a monitored target area. This can be achieved by automatically processing one or more captured images (e.g. frames of a captured video) of the target area. In particular, it is proposed to process image data of the target area using both an object detection model and a motion detection model. That is, it is proposed to acquire and use both information about an infant's breathing, and information describing the likelihood there is an infant present in an image of the target area to determine whether the infant is absent from the target area or whether the infant is present in the target area. Such a proposed approach may be less likely to produce a false detection of infant absence when compared to using an object detection model in isolation.

The first and second image data used in the disclosed method may be in any appropriate form. This may include full or partial images, or full or partial frames of video data. The first and second image data may be obtained using any suitable image sensor or camera-equipped device. For example, the first and second image data may comprise a series of cropped frames of video data from a video-based infant monitor. The cropped area may be predefined or alternatively may be selected by the user.

The proposed approach may have the additional advantage that it can be carried out using a single image sensor which may form part of any commonly-used device that has image/video capture capabilities. For example, this method could be implemented using data from known infant monitors, smartphone devices, or webcams. Embodiments may therefore leverage and/or employ existing monitoring equipment. Embodiments may also be implemented to extend the functionalities of conventional infant monitoring systems.

Proposed embodiments thus aim to provide an accurate prediction as to whether an infant is absent from or present in a target area. This prediction may then be used in an automatic sleep logging function of an infant monitoring system. Video data from an image sensor of the image monitoring system may be sampled and processed at regular time intervals, for example every second, with an AI object detection model to output an infant presence status associated with a target area present in the video data. The infant presence status may describe a confidence level associated with an infant being present in the target area. The video data may also processed at regular intervals using an actigraphy machine learning neural network model which outputs an infant breathing status which may describe a confidence level associated with there being a breathing infant present in the target area. A processing arrangement may then use these two outputs to determine whether the infant is absent from the target area. These time-sequential determinations of infant presence/absence/unknown may then be used to log the time periods during which the infant is absent from the crib. Additional data obtained from processing the image data may be used to determine other related parameters such as the quality of baby sleep. These parameters and the infant sleep log may then be displayed to the parent/carer of the infant on an output interface of the infant monitor or on a separate device.

By way of summary, an exemplary embodiment comprises the following modules:
A. Object Detection: a pretrained AI object detection model is used to detect the presence of an infant body part in an image or images of a predefined target area. The objection detection model outputs a presence status value describing the likelihood that there is an infant in the analysed image data of the target area.
B. Movement Detection: a pretrained machine learning actigraphy model is used on second image data (which may be different from or the same as the first image data processed by the object detection model) to detect movement in the target area. This movement is then analysed to determine parameters relating to the breathing of any infant present in the target area.
C. Presence/Absence Logic: based on the outputs of the object detection model and the movement detection model, presence/absence logic is employed to determine whether an infant is present or absent in the target area at the time associated with the first and second image data. Alternatively, it may be determined that based on the outputs of the two models it is not possible to determine conclusively whether the infant is present or absent in the target area.
D. Sleep Dashboard: A smart infant sleep dashboard is proposed for sending notifications. By way of example, notifications may inform the user of whether the monitored infant is absent from the cot/crib or display data indicating historic sleep periods of the infant and the quality of the sleep of the infant in these sleep periods.

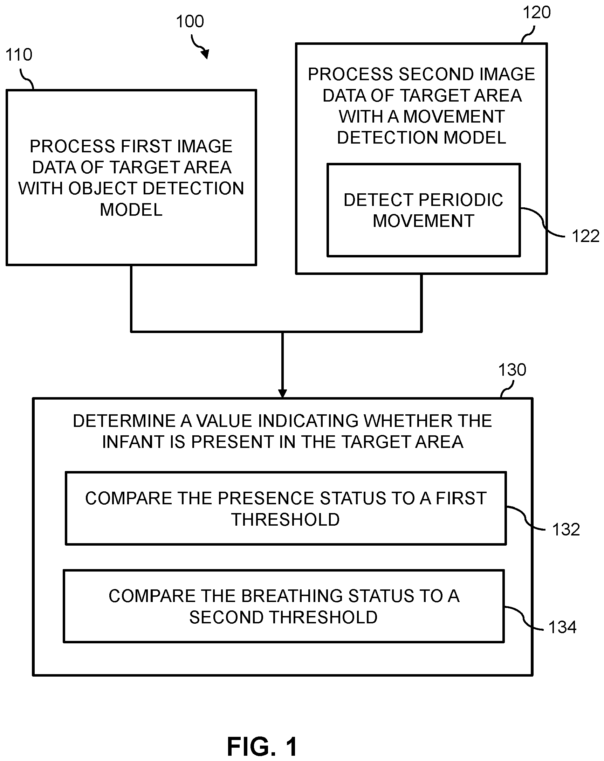

Referring now to Fig. 1, there is depicted a simplified flow diagram of a method 100 for detecting the presence or absence of an infant from a monitored target area.

The method begins with a step 110, comprising processing first image data of the target area with an object detection model, to determine a presence status of the infant, the presence status being indicative of whether the infant is in the target area. In this exemplary method the presence status comprises a probability value, based on the first image data of the target area, describing the likelihood of whether an infant is present in the target area. The first image data, in this embodiment, is a frame of a video taken by a video baby monitor.

The object detection model in this exemplary embodiment is an AI model trained to detect the presence of an infant's head in the image of the target area. Any well-known suitable object detection model may be used, for example YOLO, R-CNN, Mask R-CNN, MobileNet, or Squeeze Det. The object detection model may be a deep learning model trained on a large data set of annotated images to identify objects in an image, classify each object, and estimate the size of each object with a bounding box.

The method 100 also comprises a step 120 in which second image data of the target area is processed with a movement detection model to determine a breathing status of the infant, the breathing status describing at least one breathing parameter of the infant. In this exemplary embodiment, the at least one breathing parameter of the infant describes a probability that a breathing infant is present in the target area. The second image data in this embodiment comprises a plurality of time-sequenced images of the target area e.g., several sequential frames of a video taken by a video baby monitor. The step 120 is carried out irrespective of the determination of the object detection model in step 110. Hence, the proposed invention always requires the use of both an object detection model and a movement detection model in any determinations of infant presence or absence.

In this embodiment, the step 120 further comprises the sub-step 122. Step 122 comprises detecting periodic movement in the plurality of time-sequenced images. At least one of: the periodicity of the periodic movement; the amplitude of the periodic movement; and the regularity of the periodic movement may also be determined. Thus, the movement detection model is used to identify periodic movement in the target area, determine various characteristics of the identified periodic movement, and use this information to determine the probability of there being a breathing infant in the target area.

Once the presence status and breathing status of the infant have been determined the method proceeds to a step 130. Step 130 comprises determining, based on the determined presence status of the infant and the determined breathing status of the infant, a value indicating whether the infant is present in the target area. This value may comprise a simple determination of "present" or "absent" but may also include an indication of the probability of an infant being present or absent. Further, this value may include a determination that, based on the presence status and the breathing status, it is inconclusive as to whether the infant is in their crib or not.

In this exemplary embodiment, step 130 further comprises the sub-steps 132 and 134. Step 132 comprises comparing the determined presence status to a first threshold, and step 134 comprises comparing the determined breathing status to a second threshold. In this exemplary embodiment, the presence status comprises a probability that there is an infant present in the target area and the breathing status comprises a probability that there is a breathing infant present in the target area. Therefore, the sub-steps 132 and 134 involve comparing these probability values to certain threshold probability values. A certain decision logic will then be carried out to determine whether the infant is absent from the target area. Further, in this example the first threshold and the second threshold have different values to reflect the different levels of reliability of the two models (i.e. the object detection model and the movement detection model) being used. For example, it may be determined that the infant is absent from the target area only if the probability value of the presence status is below 0.3 and the probability value of the breathing status is below 0.4, and only determined that the infant is present if the presence status is higher than 0.6 or the breathing status is above 0.95. Many suitable logic systems may be employed to determine whether the infant is in the target area and this logic is dependent on the known capabilities of the two methods being employed. A determination of infant presence or absence may require both the presence status and the breathing status to be within a predetermined range (AND logic), or may require only one of the two values to be within the predetermined range (OR logic), but in all cases both the presence status and the breathing status are calculated before the determination is carried out.

From Fig. 1 it will be appreciated that step 110 and step 120 occur independently of one another. Crucially, the determination of the breathing status of the infant occurs irrespective of the value of the presence status. It will therefore be understood that steps 110 and 120 may occur sequentially in any order, at overlapping times, or concurrently. When steps 110 and 120 occur in parallel, the proposed method may have the advantage that the determination of infant presence or absence may be faster, which may be useful in the application of real-time monitoring of an infant's crib.

In the above outlined method, the presence status is a probability value, but the proposed invention is not limited to such. For instance, the presence status may simply be a determination of 'present' or `not present' or may comprise a plurality of different values relating to the likelihood that an infant is present in the first image data and the position of any detected body parts of the infant. The first image data may not be a frame of a video taken from a video baby monitor but may comprise an image taken by a webcam or smartphone.

Similarly, other aspects of the method outlined above may be implemented differently in alternative embodiments. For instance, the at least one breathing parameter of the infant may further describe at least one of: a probability that a breathing infant is present in the target area; a respiration rate; a respiration depth and a respiration regularity parameter. These parameters may be determined based on the characterization of the detected periodic movement in the second image data.

Any suitable image data of the target area to which motion detection models can be applied may be used as the second image data. This includes any image or images taken of the target area from any capable device. The first and second image data need not be the same and may indeed be in different forms to reflect the fact that they are being processed using different models with different data requirements. Similarly, the first and second image data may be taken by the same or different image sensors. Using the same image sensor to acquire both sets of data has the advantage that it reduces the number of sensors required to be included in a suitable monitoring device of the infant's crib and further ensures that this method can be integrated into existing infant monitoring systems. Using different image sensors may enable the first and second image data to have different view directions of the target area. This may be advantageous as the accuracy of the two models may depend differently on the view direction of the image data they are applied to.

The object detection model and the movement detection model may employ machine-learning algorithms. A machine-learning algorithm is any self-training algorithm that processes input data in order to produce or predict output data. Here, the input data comprises video or image data of a monitored area which may include an infant bed or crib and the output data comprises a presence status of the infant describing a prediction as to whether the infant is in the target area in the case of the object detection model, and a breathing status of the infant describing at least one breathing parameter of the infant in the case of the motion detection model.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. Purely by way of example, one or more of the machine learning models may employ a Convolution Neural Network, because CNNs have been proven to be particularly successful at analysing images, and being able to identify objects within images, with a much lower error rate than other types of neural network.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). Some embodiments of the present invention may employ CNN-based learning algorithms, because CNNs have proved to be particularly successful at analysing images, and are able to identify objects within images with a much lower error rate than other types of neural network.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the object detection model correspond to example images of an infant laying in a bed. The training output data entries correspond to the co-ordinates of the bounding boxes representing the spatial location of the body parts of the infant in the training image. In the case of the movement detection mode, the input data entries may include snapshots of an infant sleeping in a bed, and the training output data entries correspond to breathing parameters of the infant. In other words, the object detection model and the movement detection model may be trained using a using a deep learning algorithm configured to receive an array of training inputs and respective known outputs, wherein a training input comprises training image data of an infant laying or sleeping in a bed and a respective known outputs. In this way, the object detection model may be trained to output a prediction of the spatial location of an infant's body parts head and upper torso when provided with an image of a lying infant and the movement detection model may be trained to output at least one breathing parameter of an infant when provided with a video of a sleeping infant.

The object detection model and the movement detection model may be a pre-trained models further trained on images of a laying or sleeping infant that have been manually annotated. For example, short videos may be taken of laying infants from various angles. Images may then be extracted from the videos in order to serve as training inputs with known infant positions and know infant breathing parameters. This allows the models to become especially proficient at predicting the presence status and breathing status of the infant, regardless of image capture (i.e., camera) position.

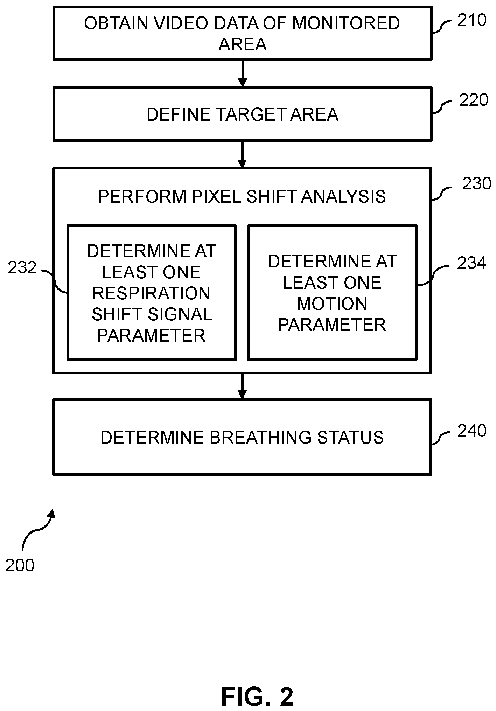

Referring now to Fig. 2 there is depicted a simplified flow diagram of a method 210 for processing image data with a movement detection model according to a proposed embodiment. This embodiment depicts in more detail how an actigraphy machine learning neural network model may be used to determine the breathing status of an infant in a target area.

The method starts with a step 210 which comprises obtaining video data of the area being monitored. This may be taken by an infant monitoring device equipped with an image sensor and positioned above the bed or crib of an infant such as to obtain a birds-eye view of the sleeping area of the infant. The video data is then provided to a processing arrangement configured to process the video data with a motion detection model.

The method then proceeds to a step 220 which comprises defining the target area to be analysed. In this exemplary embodiment, this involves cropping the total area depicted in the video data obtained in step 210, to obtain cropped video data of a smaller target area corresponding to the mattress area of the infant's bed or crib. For example, a 720p crop may be taken of a full HD video stream. The cropped target area may be selected by the user or may be preselected by the motion detection model. For the case of a user-selected target area, the size of the selected area may be equal to the size of crop required or the user may select an area that has a different size to the required crop size. In this latter case, the defined target area may be centred using the area selected by the user. The cropping step described herein reduces the computational power required to process each frame of the video data and allows the surrounding of the crib to be filtered out which may otherwise result in false motion detection signals.

Once the target area of the video data has been defined the method moves to a step 230 comprising performing pixel shift analysis on the cropped video data. This involves analysing at least two frames of the cropped video data, segmenting the cropped area into smaller blocks of, for example, 80 by 80 pixels, and identifying any texture shifts between corresponding pixel blocks in the two frames. The pixel shift analysis may be carried out on every frame of the video data, wherein the frame rate of the video data is 8 fps, 10 fps or 12 fps. The pixel shift analysis results in the detection of both periodic and non-periodic motion in the target area. The pixel shift analysis step 230 further comprises the sub-steps 232 and 234.

Step 232 comprises determining at least one respiration shift signal parameter, wherein the at least one respiration shift signal parameter describes at least one characteristic of any detected periodic motion that is likely to relate to breathing of an infant in the target area. In this exemplary embodiment, the at least one respiration shift signal parameter comprises: a shift signal amplitude describing the average amplitude of detected texture shifts, a spectral flatness parameter describing the level of noise of the detected shifts, and a breathing confidence metric describing the regularity of the periodicity of the detected periodic motion.

Step 234 involves determining at least one motion parameter describing any non-periodic motions detected in the cropped video data which may relate to an infant moving or rolling-over in the target area. In this exemplary embodiment this involves averaging the texture shifts detected across all the pixel blocks analysed. If the average texture shift exceeds a given threshold it is determined that a large motion has occurred. If any large motions have occurred in the last 30 seconds there is a high likelihood that an infant is in the target area at the present instance.

In step 240, the breathing status of the infant is determined based on the at least one respiration shift signal parameter and the at least one motion parameter.

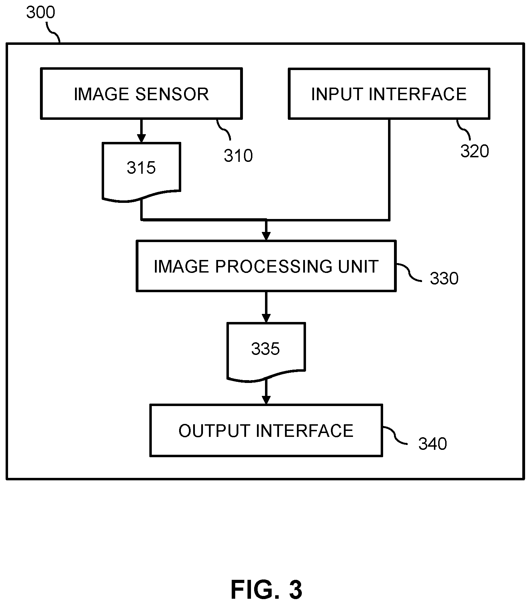

Referring now to Fig. 3, there is depicted a simplified block diagram of an infant monitoring system 300 for detecting the presence or absence of an infant from a target area being monitored by the infant monitoring system according to a proposed embodiment. The infant monitoring system comprises an image sensor 310, an input interface 320, an image processing unit 330, and an output interface 340. The image processing unit 330 may also be referred to as a processor arrangement.

The system 300 is configured to determine the likelihood of an infant being present or absent from a target area being monitored by the system. It achieves this by processing in the image processing unit 330 image data from the image sensor 310. This may be carried out using the method 100.

In detail, the image sensor 310 produces image data 315 of an area to be monitored. The input interface 320 permits a user to select a smaller target area from the total area being monitored by the image sensor. For example, a user of the infant monitoring system may select via the input interface the area being monitored that corresponds to the mattress area of an infant's crib. The selected target area along with the image data from the image sensor may be sent to the image processing unit 330. The image data 315 may comprise a first image data and a second image data.

The image processing unit 330 is configured to run computer code comprising code means for implementing the method 100. Thus, on receiving the image data 315 the image processing unit 330 processes the first image data with an object detection model to determine a presence status of the infant, the presence status being indicative of whether the infant is present in the target area. The processing unit then processes the second image data with a movement detection model to determine a breathing status of the infant. The processor then uses a presence/absence logic system to determine, based on the determined presence status of the infant and the determined breathing status of the infant, a value indicating whether the infant is present in the target area.

The image processing unit 330 is configured to output an indication 335 of whether the infant is absent area from the target area to the output interface 340 which then may output this indication to the user (e.g. as an electronic output signal and/or a visible/audible output).

The output interface 340 may be further configured to output more detailed information describing an infant's sleep to aid parents in assessing and monitoring the sleep of their infant. The method 100 may be performed on image data taken at regular time intervals to determine at regular time intervals the likelihood of the infant being absent from the target area. These determinations may then be used to automatically log the periods for which the infant is absent from the bed which may then be displayed to the parent/carer on the output interface. The presence/absence determination may further be used to determine other aspects of the output interface 340. For example, if it is determined that there is a high likelihood that the infant is absent from the target area, the output interface may not display sleep data to the user, and in this way the system may save power. Other data, such as the determined breathing parameters and/or motion parameters, obtained via the processes outlined in the above embodiments may also be displayed by the output interface.

For example, the output interface may be configured to display a sleep dashboard to the user. The dashboard may include a live video of an infant lying in the crib. The video streaming can be turned on or off depending on the requirements of the parents and the resources available in the device. Several features related to different aspects of infant sleep may be tracked and communicated by the dashboard in real time such as: whether infant is present in crib; infant awake or asleep status; infant breathing status (if yes, an indication of whether breathing is within normal range may also be provided); and audible and/or visible alert(s) if the infant is detected to be absent and/or when there is an anomaly in breathing rate.

The dashboard may thus provide high-level analytics/insights regarding the infant's sleep habits (based on the sleep history for example). For example, a bar chart of total sleep duration in past seven days may be displayed to a user. Such data can support the parents in determining if the sleep cycle of the infant is being affected when there is an anomaly in the chart. The bar chart may also extend to providing the sleep duration in past two weeks or past month.

Sleep status reports describing sleep data (such as the average, maximum and minimum number of hours an infant sleep daily, etc.) may also be provided by the dashboard. Furthermore, a sleep interruption report may also be provided which includes information on whether any interruption occurred during sleep. The report may track the average number of interruptions occurring daily during sleep, when does most sleep interruptions happen - daytime or at night, and the potential cause of sleep interruption - external noise, abnormal breathing, crying, etc. Also, a sleep ambient report may be provided which includes information describing the average temperature and humidity of the room where the infant lies in the crib. Finally, a breathing rate report may be provided which tracks the breathing activity of the infant in real time and an alert may be triggered to the parents when an anomaly in infant breathing is detected.

Although in the system 300, the image sensor 310, the input interface 320, the image processing unit 330, and the output interface 340 are present in the same device, it will be understood that these components may be distributed among various devices. For example, there may be provided an infant monitor comprising only an image sensor. The image data taken by the image sensor of the image monitor may then be relayed to a personal smart device of the parent/carer. The processing and interface capacities of the personal smart device may then fulfil the functionalities of the input interface 320, image processing unit 330, and the output interface 340.

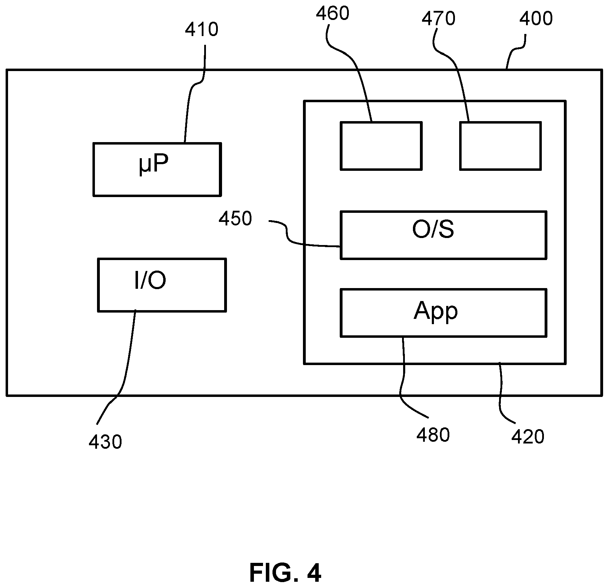

Fig. 4 illustrates an example of a computer 400 within which one or more parts of an embodiment may be employed. Various operations discussed above may utilize the capabilities of the computer 400. In this regard, it is to be understood that system functional blocks can run on a single computer or may be distributed over several computers and locations (e.g. connected via internet).

The computer 400 includes, but is not limited to, PCs, workstations, laptops, PDAs, palm devices, servers, storages, and the like. Generally, in terms of hardware architecture, the computer 400 may include one or more processors 410, memory 420 and one or more I/O devices 430 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), or an auxiliary processor among several processors associated with the computer 400, and the processor 410 may be a semiconductor-based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 450, compiler 460, source code 470, and one or more applications 480 in accordance with exemplary embodiments. As illustrated, the application 480 comprises numerous functional components for implementing the features and operations of the exemplary embodiments. The application 480 of the computer 400 may represent various applications, computational units, logic, functional units, processes, operations, virtual entities, and/or modules in accordance with exemplary embodiments, but the application 480 is not meant to be a limitation.

The operating system 450 controls the execution of other computer programs, and provides scheduling, input-output control, file and data management, memory management, and communication control and related services. It is contemplated by the inventors that the application 480 for implementing exemplary embodiments may be applicable on all commercially available operating systems.

Application 480 may be a source program, executable program (object code), script, or any other entity comprising a set of instructions to be performed. When a source program, then the program is usually translated via a compiler (such as the compiler 460), assembler, interpreter, or the like, which may or may not be included within the memory 420, so as to operate properly in connection with the O/S 450. Furthermore, the application 480 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 430 may include input devices such as, for example but not limited to, a mouse, keyboard, scanner, microphone, camera, etc. Furthermore, the I/O devices 430 may also include output devices, for example but not limited to a printer, display, etc. Finally, the I/O devices 430 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 430 also include components for communicating over various networks, such as the Internet or intranet.

If the computer 400 is a PC, workstation, intelligent device or the like, the software in the memory 420 may further include a basic input output system (BIOS) (omitted for simplicity). The BIOS is a set of essential software routines that initialize and test hardware at start-up, start the O/S 450, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 410 is configured to execute software stored within the memory 420, to communicate data to and from the memory 420, and to generally control operations of the computer 400 pursuant to the software. The application 480 and the O/S 450 are read, in whole or in part, by the processor 410, perhaps buffered within the processor 410, and then executed.

When the application 480 is implemented in software it should be noted that the application 480 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 480 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-2, and the system of Fig. 3, may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs. 3 and 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams, and combinations of blocks in the block diagrams and/or flow diagrams, can be implemented by special purpose hardware-based systems that perform the specified functions or acts or carry out combinations of special purpose hardware and computer instructions the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams, and combinations of blocks in the block diagrams and/or flow diagrams, can be implemented by special purpose hardware-based systems that perform the specified functions or acts or carry out combinations of special purpose hardware and computer instructions.

## Claims

1. A method (100) for detecting the presence or absence of an infant from a monitored target area, wherein the method comprises:
processing (110) first image data of the monitored target area with an object detection model to determine a presence status of the infant, the presence status being indicative of whether the infant is present in the target area;
independent of processing the first image data, processing (120) second image data of the monitored target area with a movement detection model to determine a breathing status of the infant, the breathing status describing at least one breathing parameter of the infant; and
determining (130), based on the determined presence status of the infant and the determined breathing status of the infant, a value indicating whether the infant is present in the target area.

2. The method of claim 1, wherein the presence status comprises a probability value.

3. The method of any of claims 1-2, wherein the at least one breathing parameter of the infant describe at least one of: a probability that a breathing infant is present in the target area; a respiration rate; respiration depth; and a respiration regularity parameter.

4. The method of any of claims 1-3, wherein determining a value indicating whether the infant is present in the target area comprises:
comparing (132) the presence status to a first threshold;
comparing (134) the breathing status to a second threshold; and
determining (130), based on the comparison of the presence status to the first threshold and the breathing status to a second threshold, a likelihood of the infant being absent from the target area.

5. The method of any of claims 1-4, wherein the object detection model is trained to detect at least one body part of the infant.

6. The method of any of claims 1-5, wherein the second image data of the monitored target area comprises a plurality of time-sequenced images.

7. The method of any of claims 1-6, processing second image data of the monitored target area with a movement detection model comprises detecting periodic movement.

8. The method of claim 7, wherein detecting periodic movement further comprises determining at least one of: the periodicity of the periodic movement; the amplitude of the periodic movement; and the regularity of the periodic movement.

9. The method of any of claims 1-8, wherein the first image data and the second image data are from a single image sensor.

10. A computer program comprising code means for implementing the method of any preceding claim when said program is run on a processing system.

11. A processor arrangement (410) comprising the computer program of claim 9 and configured to execute the computer program.

12. A system (300) for detecting the presence or absence of an infant from a monitored target area, the system comprising:
an image processing unit (330) configured to run the computer program of claim 10.

13. An infant monitoring system, comprising:
the system of claim 12; and
at least one of:
an output interface (340) configured to output an indication of whether an infant is present or absent from the target area;
an input interface (320) configured to permit to user of the infant monitoring system to select the target area; and
an image sensor (310).
